# EUROPÄISCHE PATENTSCHRIFT

(11) **EP 0 394 787 B1**
(45) Veröffentlichungstag und Bekanntmachung des Hinweises auf die Patenterteilung: **08.06.1994**
(21) Anmeldenummer: 90107136.5
(22) Anmeldetag: 13.04.1990
(51) Int. Cl.: C07C 233/20, C07C 233/18, C08F 20/36, C09J 4/02, A61K 6/00, A61L 25/00

(54) **Zubereitungen enthaltend Carbonamidgruppen enthaltende (Meth)acrylsäureester, zur Anwendung als Adhäsivkomponenten zur Behandlung kollagenhaltiger Materialien in der Medizin, sowie Herstellung dieser Zubereitungen**
Compositions containing carbamide group containing (meth)acrylic acid esters for use as adhesive components for the treatment of collagenic materials in medicine, and preparation of the compositions
Compositions contenant esters de l'acide (méta)acrylique contenant des groupes carbamides pour utilisation comme composants adhésifs pour le traitement de matériaux collagènes médicaux, et préparation des compositions

(30) Priorität: 27.04.1989 DE 3913939
(43) Veröffentlichungstag der Anmeldung: 31.10.1990
(73) Patentinhaber: BAYER AG, 51368 Leverkusen (DE)
(72) Erfinder: Müller, Michael, Dr., D-5060 Bergisch-Gladbach 2 (DE); Podszun, Wolfgang, Dr., D-5000 Köln 80 (DE); Alker, Bernd, Dipl.-Ing., D-5060 Bergisch Gladbach 2 (DE)

(56) Entgegenhaltungen:
- EP-A- 0 141 324
- EP-A- 0 264 551
- EP-A- 0 326 132
- EP-A- 0 355 562
- FR-A- 2 301 539
- GB-A- 2 009 745
- US-A- 3 366 613

## Beschreibung

Die Erfindung betrifft Zubereitungen zur Anwendung als Adhäsivkomponente zur Behandlung kollagenhaltiger Materialien in der Medizin, die Verbindungen (I) enthalten und Verfahren zur Herstellung der Zubereitungen.

Die beanspruchten Zubereitungen enthalten Carbonamidgruppen enthaltende (Meth)acrylsäureester (I) der Formel 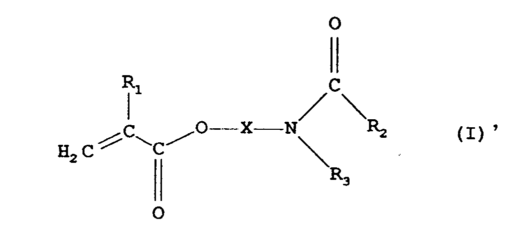
in der
- R₁: Wasserstoff oder Methyl bedeutet,
- R₂: gegebenenfalls durch Hydroxy, Carboxy, Halogen oder Amino der Formel 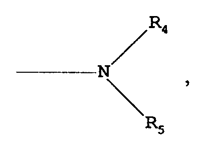 in der
- R₄ und R₅: gleich oder verschieden sind und Wasserstoff oder Niederalkyl bedeuten,
substituiertes Alkyl (C₁-C₄) oder Alkenyl (C₂-C₄) bedeutet,
- R₃: Wasserstoff bedeutet oder eine der oben für R₂ genannten Bedeutungen hat und
- X: ein zweiwertiger, gegebenenfalls durch Hydroxy, Carboxy, Halogen oder Amino der Formel 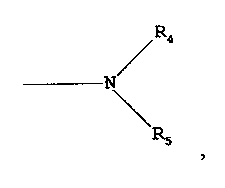 in der
- R₄ und R₅: die oben genannte Bedeutung haben,
substituierter aliphatischer (C₁-C₆) oder cycloaliphatischer Rest (C₃-C₆), der gegebenenfalls eine oder mehrere Sauerstoff-, Schwefel- und/oder NR₄-Brücken enthalten kann, wobei
- R₄: die oben genannte Bedeutung hat,
ist, mit der Maßgabe, daß
a) die Summe der Kohlenstoffatome in den Resten R₂, R₃ und X nicht größer als sechs ist, sofern diese Reste keine Heteroatome enthalten, oder
b) die Summe der Kohlenstoffatome in den Resten R₂, R₃, R₄, R₅ und X nicht größer als zehn ist, sofern mindestens einer dieser Reste zumindest ein Heteroatom enthält,
und gegebenenfalls Initiatoren.

Carbonamidgruppen enthaltende (Meth)acrylsäureester sind teilweise aus der US-A-3,366,613, GB-A-2 009 745 und EP-A-0 326 132 bekannt. Erfindungsgemäße Carbonamidgruppen enthaltende (Meth)acrylsäuerester (I) können durch die Umsetzung von Alkanolaminen mit Carbonsäureestern und (Meth)acrylsäurechlorid hergestellt werden.

In der JP 47-36265 (72-36265) sind ähnliche Verfahren für die Herstellung von (Meth)acrylsäureester-Derivaten beschrieben.

Kollagenhaltige Materialien sind Gerüsteiweißkörper und Hauptbestandteile der menschlichen und tierischen interzellularen Stützsubstanzen wie Knorpel- und Knochengewebe, Haut und Zahnbein (Dentin). Im Rahmen der vorliegenden Erfindung werden die Adhäsivkomponenten bevorzugt zur Behandlung von Dentin im Zusammenhang mit Zahnreparaturen verwendet.

Besonders im Dentalbereich werden härtende, polymere Materialien als Füllmaterialien bei Zahnreparaturen verwendet. Alshärtende, polymere Materialien werden im allgemeinen Füllungen auf Acrylatbasis bevorzugt. Diese polymeren Füllungen haben jedoch den Nachteil, daß sie schlecht am Dentin haften bleiben. Um dieses Problem zu lösen, hat man bisher teilweise Unterschneidungen am Zahnbein vorgenommen; dazu war es erforderlich, über den angegriffenen Bereich hinaus, beachtliche Mengen an frischem Dentin zu entfernen.

Nach einer anderen Methode ätzt man das Dentin und die Schmelzoberfläche mit Säuren, wie z.B. Phosphorsäure, an, und nimmt dann die Füllung vor. Abgesehen davon, daß die Säure eine Reizwirkung im Mundbereich ausübt, dringt sie auch leicht durch die Dentinkanäle in den Zahn und schädigt den Nerv (Pulpa).

In J. Dent. Res. 57, 500-505 (1978), werden aldehydgruppenhaltige Methacrylate der isomeren Hydroxybenzaldehyde beschrieben, die als Grundierungsmittel für Füllungen im Dentalbereich verwendet werden können. Die Bindung zwischen Dentin und Füllmasse bleibt jedoch auch nach einer solchen Grundierung unbefriedigend.

In Scand. J. Dent. Res. 92, 980-983 (1948) und J. Dent. Res. 63, 1087-1089 (1984) werden Grundierungsmittel aus wäßrigem Formaldehyd oder Glutaraldehyd und β-Hydroxyethylmethacrylat (HEMA) beschrieben.

Außerdem sind in der EP-A 0 141 324 Zusammensetzungen aus einem Aldehyd und einem olefinisch ungesättigten Monomer mit aktivem Wasserstoff beschrieben, die eine gute Anbindung an Dentin aufweisen.

Die neuen Zubereitungen auf Basis aliphatischer, Carbonamidgruppen enthaltender (Meth)acrylsäureester (I), bewirken eine starke Verbundhaftung von Materialien, die am Kollagen befestigt werden sollen, z.B. eine Verbundhaftung von Zahnfüllungsmaterial in einer Kavität am Zahn.

Methacrylsäure-2-carbonamidoethylester sind zur Herstellung wasserlöslicher Polymere aus JP 47/36265 [72/36265] bekannt. Ein aromatischer, eine Acetamidgruppe enthaltender Methacrylsäureester wurde nach Polymerisation als Proteinmodell verwendet (A. Pleurdeav et. al Eur. Polym. J. 18 (1982), 627).

Formamidgruppen enthaltende (Meth)-acrylsäureester sind aus der DE-A-2 507 189 bekannt. In der DE-A-2 507 189 wird auch beschrieben, diese Acrylsäureester als Überzüge oder Klebemittel für Papier und Textilien zu verwenden.

Die Verwendung der erfindungsgemäßen Carbonamidgruppen enthaltenden (Meth)-acrylsäureester (I) als Adhäsivkomponente für kollagenhaltige Materialien war überraschend, weil sie keine reaktiven Gruppen enthalten, die unter milden Bedingungen geeignete chemische Bedingungen zu kollagenhaltigen Materialien aufbauen können.

(Meth)-acrylsäureester im Rahmen der vorliegenden Erfindung sind die Ester der Acrylsäure und der Methacrylsäure.

Die Substituenten der erfindungsgemäßen (Meth)-acrylsäureester im Rahmen der allgemeinen Formel (I) haben im allgemeinen die folgende Bedeutung:
Halogen bedeutet im allgemeinen Fluor, Chlor, Brom und Iod, bevorzugt Fluor und Chlor.

Niederalkyl (R⁴ und R⁵) im Rahmen der Aminogruppen bedeutet im allgemeinen einen geradkettigen oder verzweigten Kohlenwassertoffrest mit 1 bis 4 Kohlenstoffatomen. Bevorzugt ist Methyl und Ethyl.

Als Aminogruppen seien beispielsweise Amino, Dimethylamino, Diethylamino, Methyl-ethyl-amino,
genannt.

Alkyl (R², R³) bedeutet im allgemeinen einen gesättigten, Alkenyl einen olefinisch ungesättigten, geradkettigen oder verzweigten Kohlenwasserstoffrest mit 1 (bzw. 2) bis 4 Kohlenstoffatomen. Beispielsweise seien die folgenden Alkylreste und Alkenylreste genannt: Methyl, Ethyl, Propyl, Isopropyl, Butyl, Isobutyl und Propenyl. Insbesondere bevorzugt werden Methyl und Ethyl.

Ein zweiwertiger aliphatischer Rest (X) bedeutet im allgemeinen einen zweiwertigen, geradkettigen oder verzweigten Kohlenwasserstoffrest mit 1 bis 6, bevorzugt 1 bis 3, Kohlenstoffatomen.

Beispielsweise seien die folgenden zweiwertigen aliphatischen Reste genannt:
Hexandiyl, Pentandiyl, Neopentandiyl, Butandiyl, Dimethylethandiyl, Propandiyl, Ethandiyl.

Bevorzugt werden als zweiwertige aliphatische Reste Ethandiyl und Propandiyl.

Ein zweiwertiger cycloaliphatischer Rest (X) bedeutet im allgemeinen einen cyclischen Kohlenwasserstoffrest mit 4 bis 6 Kohlenstoffatomen. Beispielsweise seien die folgenden zweiwertigen cycloaliphatischen Reste genannt: Cyclobutandiyl, Cyclopentandiyl, Cyclohexandiyl.

Die zweiwertigen aliphatischen und cycloaliphatischen Reste X können durch Hydroxy, Carboxy, Halogen oder Aminogruppen substituiert sein. Bevorzugt werden als Substituenten Hydroxy, Carboxy, Fluor, Chlor und Amino. X kann im allgemeinen durch 1 bis 4 Reste substituiert sein.

Es ist aber auch möglich, daß in X die aliphatischen Reste durch Sauerstoff, Schwefel und/oder -NR⁴-Brücken (wobei R⁴ die oben genannte Bedeutung hat) verbunden sind. Hierbei ist es möglich, daß die aliphatischen Reste jeweils nur durch Sauerstoff oder Schwefel oder -NR⁴ verbunden sind. Es ist aber auch möglich, daß verschiedene Brückenglieder die aliphatischen Reste miteinander verbinden.

Bevorzugt werden zweiwertige Reste, bei denen Ethylenreste über Sauerstoffbrücken gebunden sind.

Beispielsweise seien die folgenden Carbonamidgruppen enthaltenden (Meth)acrylsäureester genannt: 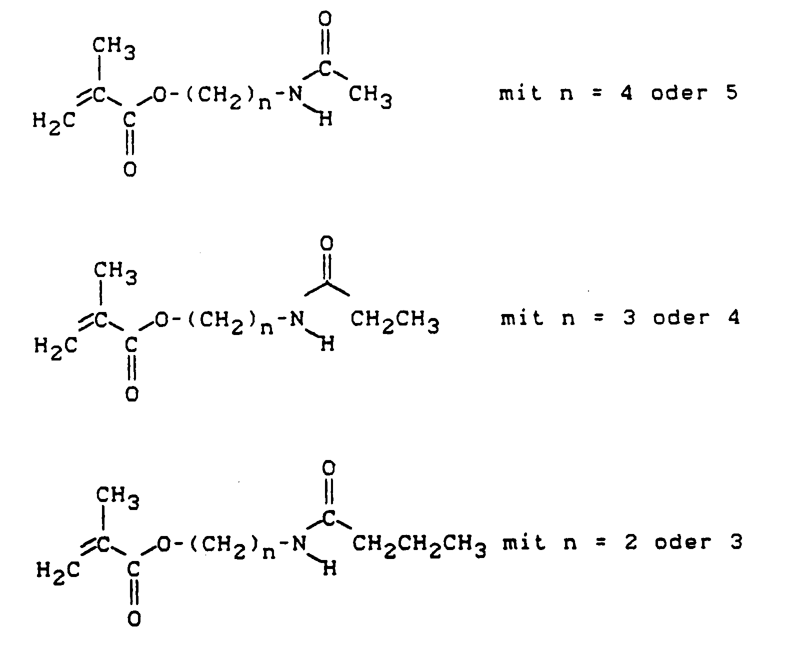 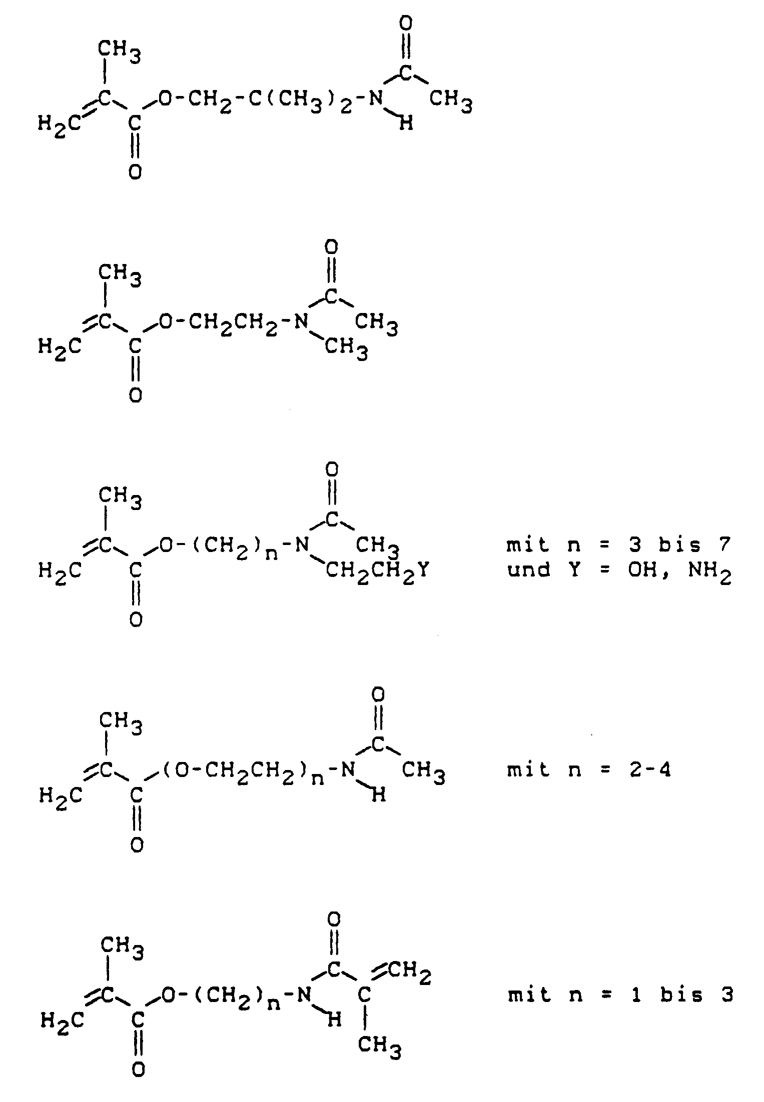
Besonders bevorzugt sind Methacrylsäure-3-acetamidopropylester und Methacrylsäure-2-acetamidoethylester der Formel 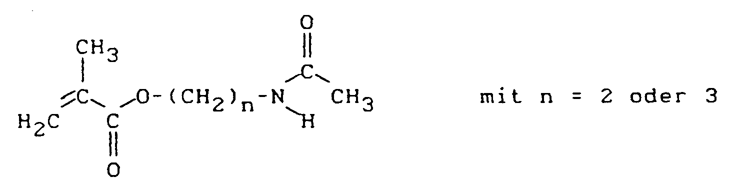
sowie Methacrylsäure-2-(N-2-hydroxyethylacetamido)-ethylester der Formel 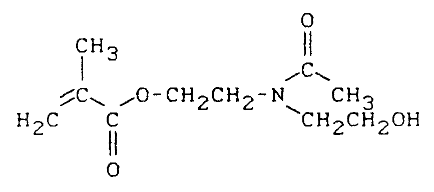
Initiatoren im Rahmen der vorliegenden Erfindung sind Radikalbildner, die eine radikalische Polymerisation auslösen. Bevorzugt sind Fotoinitiatoren, die unter der Einwirkung von Licht, beispielsweise UV-Licht, sichtbarem Licht oder Laserlicht, eine radikalische Polymerisation auslosen.

Die sogenannten Fotopolymerisationsinitatoren sind an sich bekannt (Houben-Weyl, Methoden der organischen Chemie, Band E 20, Seite 80 ff, Georg Thieme Verlag Stuttgart 1987). Vorzugsweise handelt es sich um Mono-oder Dicarbonylverbindungen, wie Benzoin und dessen Derivate, insbesondere Benzoinmethylether, Benzil und Benzilderivate, beispielsweise 4,4-Oxidibenzil und andere Dicarbonylverbindungen wie Diacetyl, 2,3-Pentandion und α-Diketo-Derivate des Norbornans und substituierter Norbornane, Metallcarbonyle wie Pentacarbonylmangan oder Chinone wie 9,10-Phenanthrenchinon und Naphthochinon. Besonders bevorzugt ist Campherchinon.

Die erfindungsgemäßen Zubereitungen enthalten im allgemeinen 0,01 bis 2 Gew.-Teile, bevorzugt 0,1 bis 0,5 Gew.-Teile des Initiators, bezogen auf 1 Gew.-Teil des Carbonamidgruppen enthaltenden (Meth)-acrylsäureesters. Enthält eines der mit der erfindungsgemäßen Adhäsivkomponente im Kontakt stehenden Fügeteile bereits einen Initiator der beschriebenen Art, kann auf den Initiator in der Adhäsivkomponente auch ganz verzichtet werden.

Die Lösungsmittel im Rahmen der vorliegenden Erfindung sollen die Komponente lösen und sollen, durch die Anwendung bedingt, untoxisch sein. Bevorzugt seien Wasser und flüchtige organische Lösungsmittel wie Methanol, Ethanol, Propanol, Isopropanol, Aceton, Methylethylketon, Tetrahydrofuran, Essigsäuremethyl- oder -ethylester genannt.

Im allgemeinen setzt man 10 bis 1000 Gew.-Teile, bevorzugt 50 bis 300 Gew.-Teile des Lösungsmittels, bezogen auf den Carbonamidgruppen enthaltenden (Meth)-acrylsäureester, ein.

Es kann vorteilhaft sein, den erfindungsgemäßen Zubereitungen Coaktivatoren zuzusetzen, die die Polymerisationsreaktion beschleunigen. Bekannte Beschleuniger sind beispielsweise Amine wie p-Toluidin, Dimethyl-p-toluidin, Trialkylamine wie Trihexylamin, Polyamine wie N,N,N′,N′-Tetraalkylalkylendiamin, Barbitursäure und Dialkylbarbitursäure.

Die Coaktivatoren werden im allgemeinen in einer Menge von 0,02 bis 4 Gew.-%, bevorzugt 0,2 bis 1 Gew.-%, bezogen auf die Menge an polymerisierbaren Verbindungen eingesetzt.

Als weitere Komponente können die erfindungsgemäßen Zusammensetzungen Carbonylverbindungen enthalten.

Carbonylverbindungen im Rahmen der vorliegenden Erfindung sind Aldehyde und Ketone, die 1 bis 20, bevorzugt 1 bis 10, und besonders bevorzugt 2 bis 6 Kohlenstoffatome, enthalten. Die Carbonylfunktion kann an einem aliphatischen, aromatischen und heterocyclischen Molekülteil gebunden sein.

Als Aldehyde seien aliphatische Mono- oder Dialdehyde genannt. Bevorzugt wird Formaldehyd, Acetaldehyd, Propionaldehyd, 2-Methylpropionaldehyd, Butyraldehyd, Benzaldehyd, Vanillin, Furfural, Anisaldehyd, Salicylaldehyd, Glyoxal, Glutardialdehyd und Phthaldialdehyd. Besonders bevorzugt ist der Glutardialdehyd.

Als Ketone seien besonders aliphatische Mono- und Diketone genannt. Bevorzugt sind Butanon, Aceton, Cyclooctanon, Cycloheptanon, Cyclohexanon, Cyclopentanon, Acetophenon, Benzophenon, 1-Phenyl-2-propanon, 1,3-Diphenyl-2-propanon, Acetylaceton, 1,2-Cyclohexandion, 1,2-Cyclopentandion und Campherchinon. Besonders bevorzugt ist Cyclopentanon.

Im allgemeinen setzt man 1 bis 1000 Gew.-Teile, bevorzugt 5 bis 50 Gew.-Teile der Carbonylverbindungen, bezogen auf den Carbonamidgruppen enthaltenden (Meth)-acrylsäureester, ein.

Als weitere Komponente können die erfindungsgemäßen Zusammensetzungen (Meth)-acrylsäureester enthalten, die Vernetzungen ausbilden können. (Meth)-acrylsäureester, die Vernetzungen ausbilden können, enthalten im allgemeinen 2 oder mehr polymerisierbare aktive Gruppen im Molekül. Bevorzugt seien Ester der (Meth)-acrylsäure mit 2- bis 5-wertigen Alkoholen mit 2 bis 30 Kohlenstoffatomen genannt. Besonders bevorzugt werden Alkoxy(meth)acrylate und Urethangruppen enthaltende (Meth)acrylate.

Beispielsweise seien (Meth)acrylsäureester der Formel 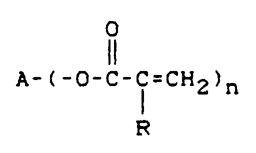
in der
- A: einen geradkettigen, verzweigten, cyclischen, aliphatischen, aromatischen oder gemischt aliphatischaromatischen Rest mit 2 bis 25 C-Atomen, der durch -O-, NH- oder O-CO-NH-Brücken unterbrochen und mit Hydroxy, Oxy, Carboxy, Amino oder Halogen substituiert sein kann,
bedeutet,
- R: H oder Methyl bedeutet und
- n: für eine ganze Zahl von 2 bis 8, vorzugsweise 2 bis 4, steht,
genannt.

Vorzugsweise seien Verbindungen der folgenden Formeln genannt: 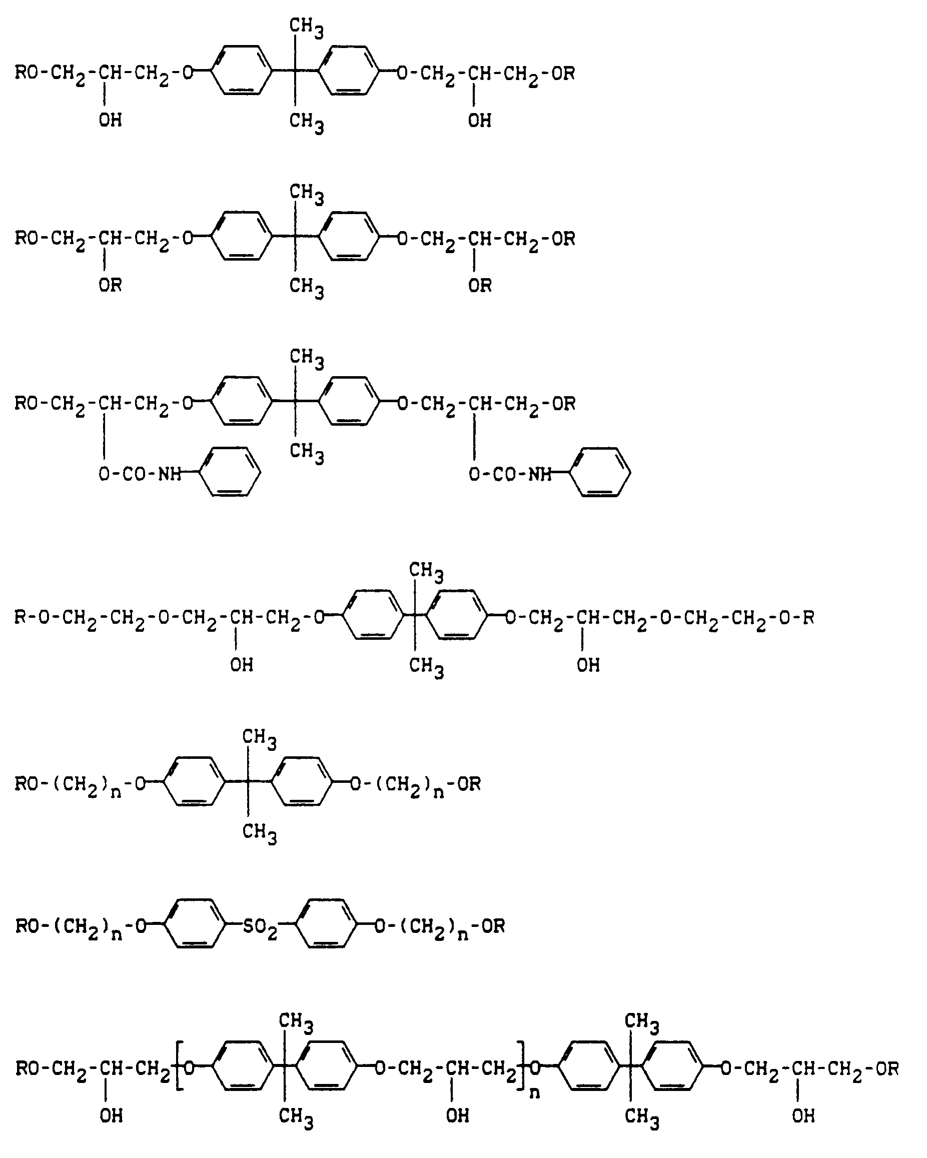 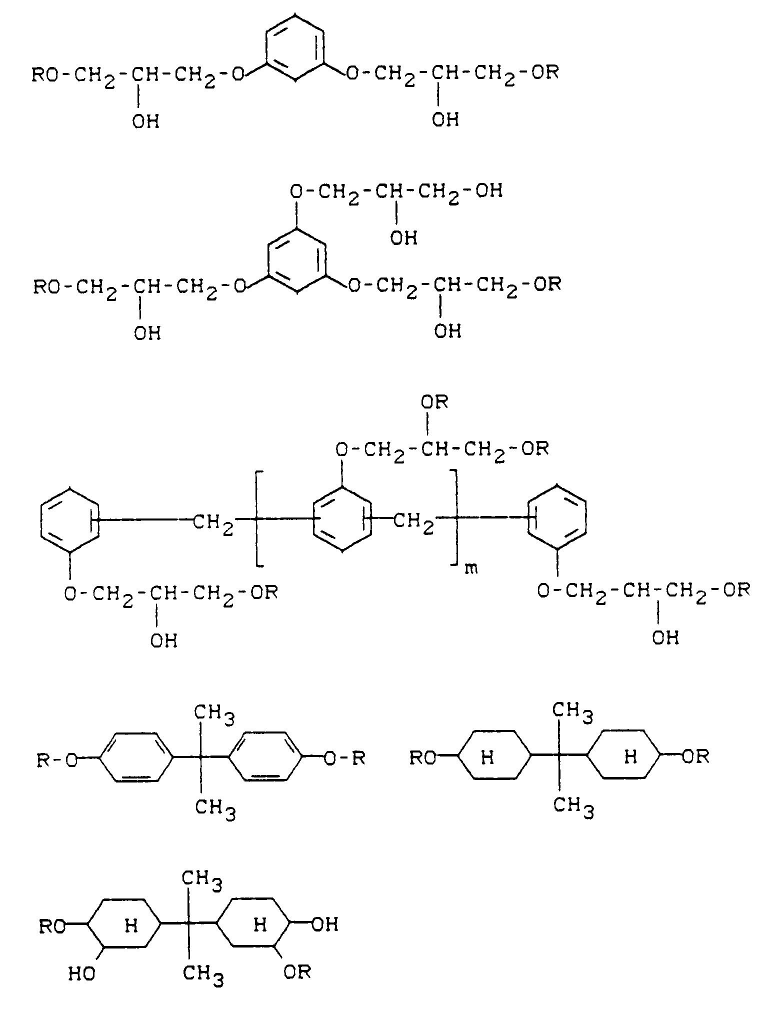 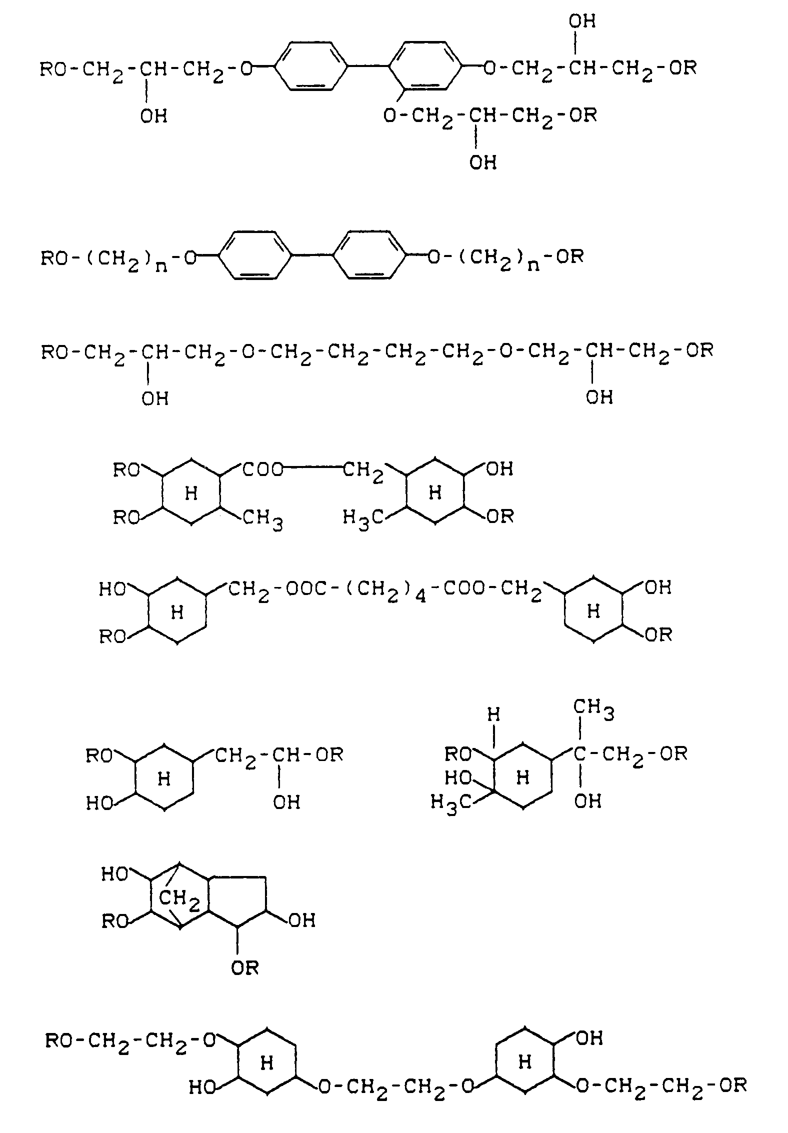 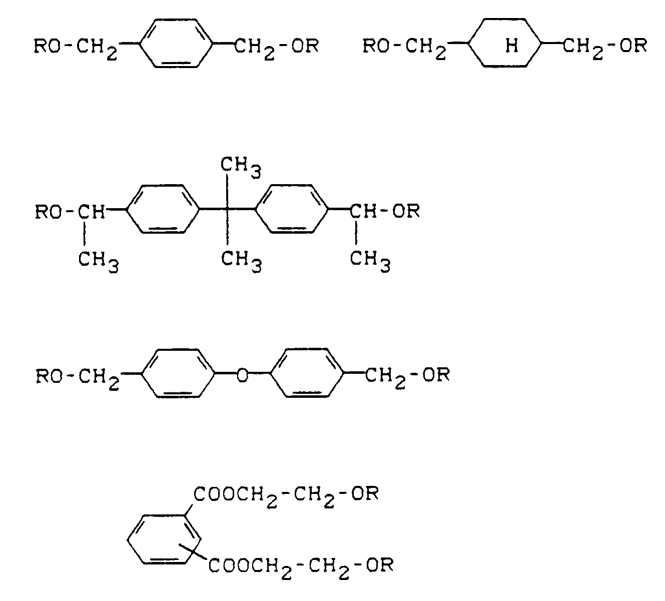
in der ortho-, meta- oder para-Form 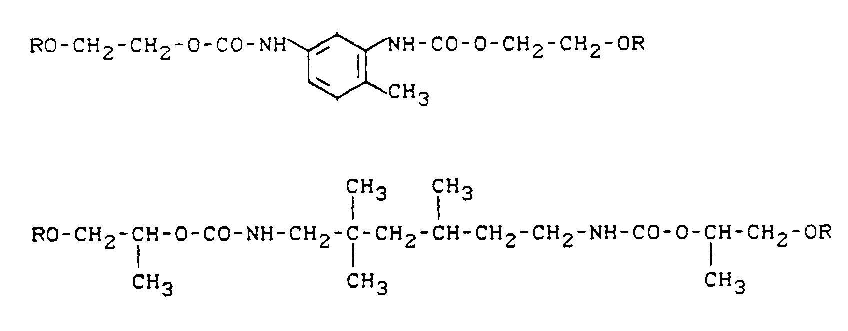 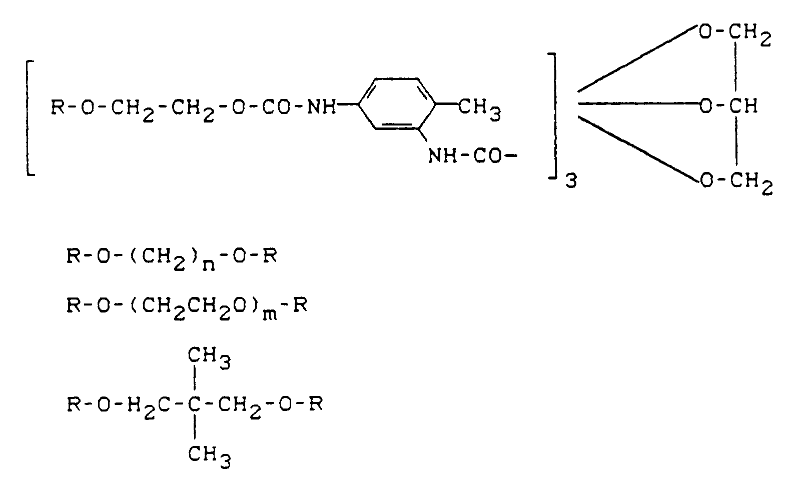
worin
- R: für 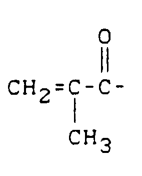 oder 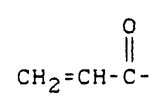 steht,
- n: eine Zahl von 1 bis 4 und
- m: eine Zahl von 0 bis 5 bedeutet.

Außerdem seien Derivate des Tricyclodecans (EP-A-00 23 686) und Umsetzungsprodukte aus Polyolen, Diisocyanaten und Hydroxyalkylmethacrylaten (DE-A-37 03 120, DE-A-37 03 080 und DE-A-37 03 130) genannt. Beispielsweise seien die folgenden Monomeren genannt; 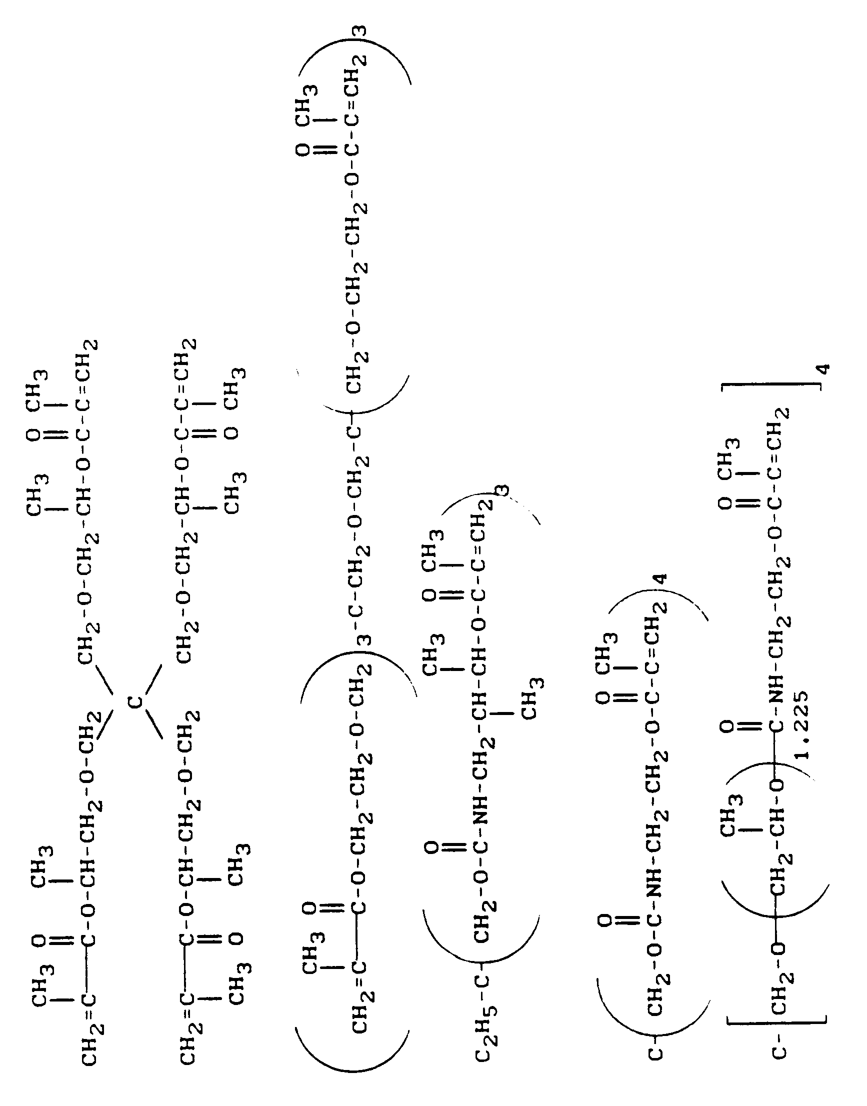 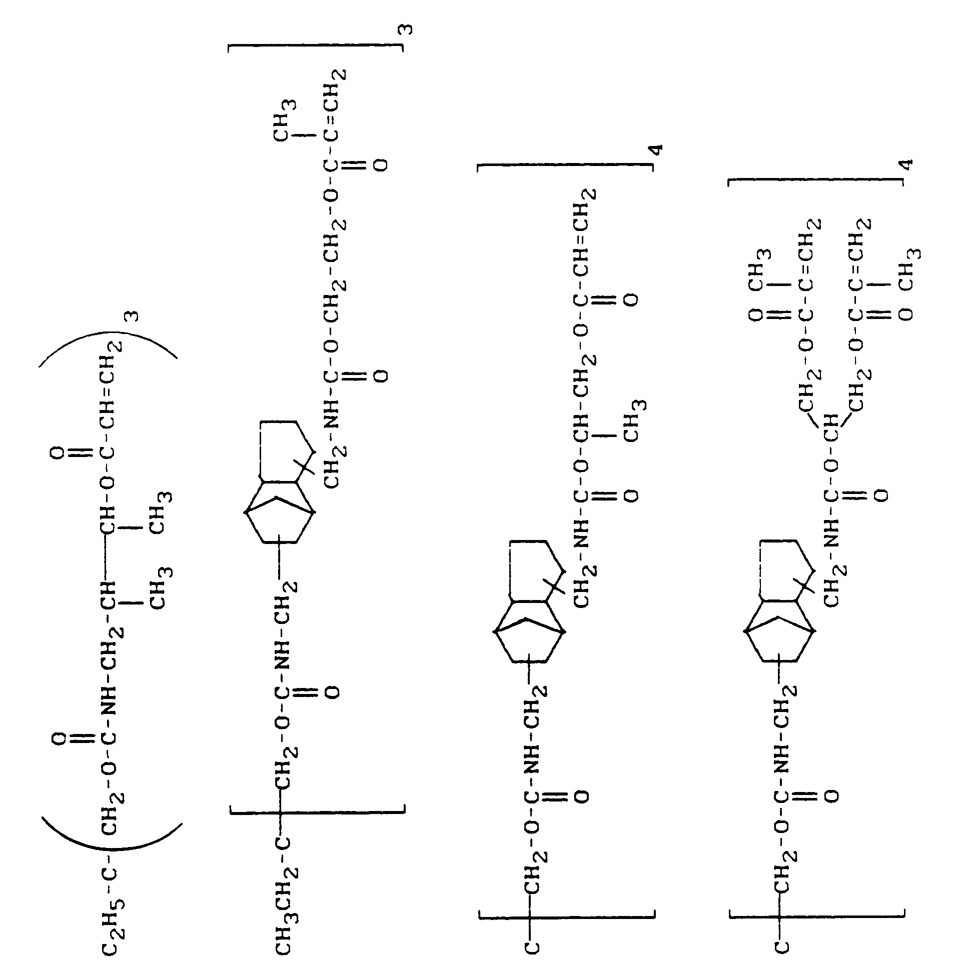 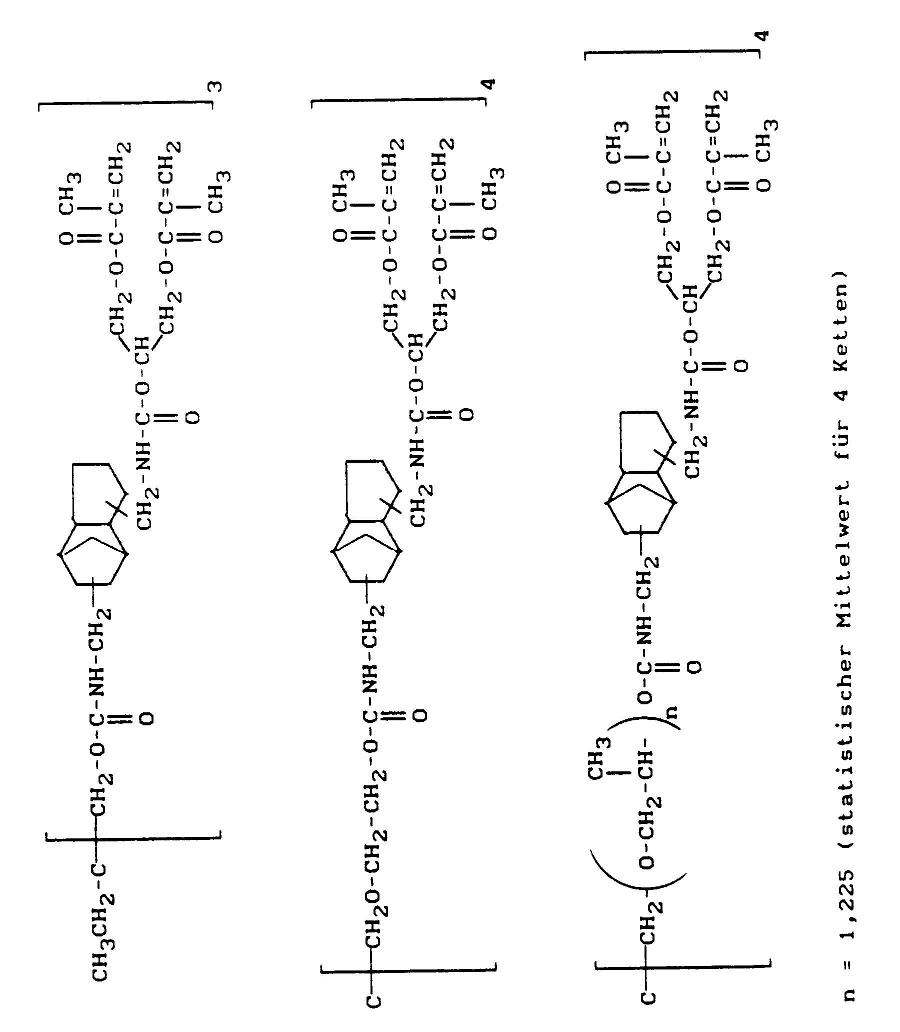 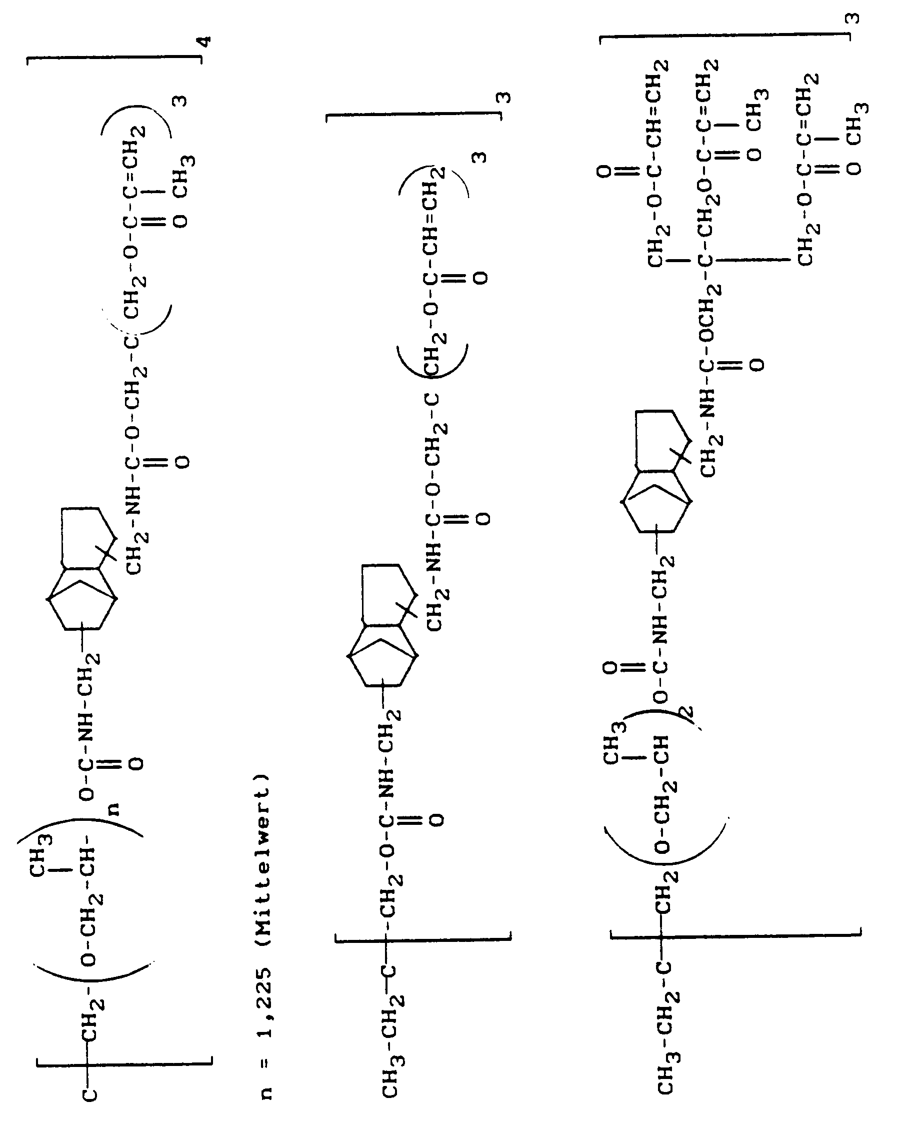 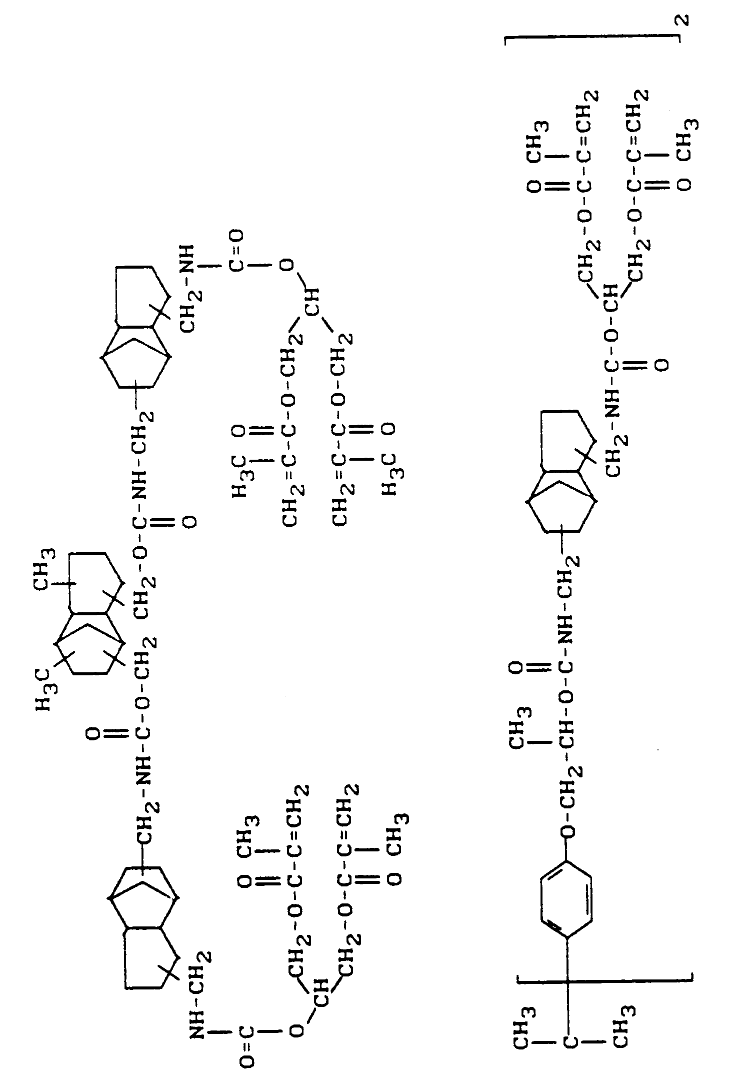 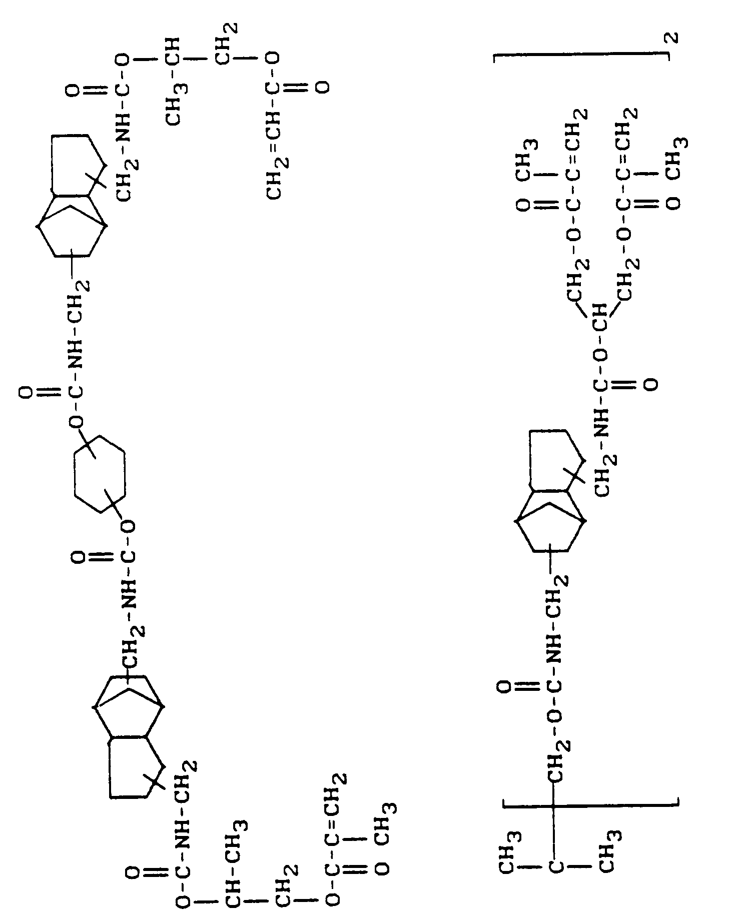 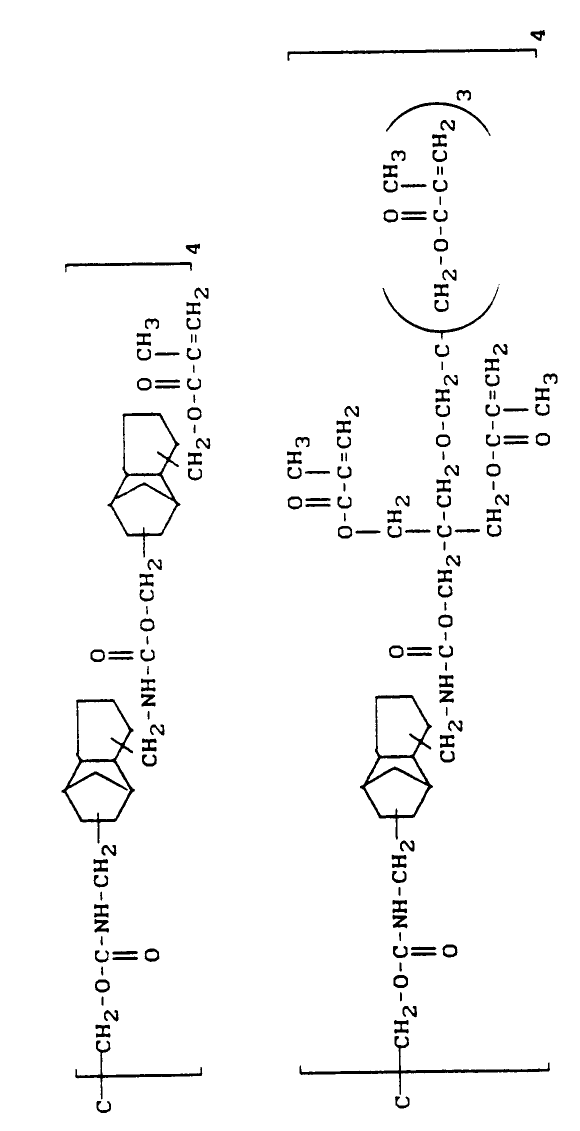 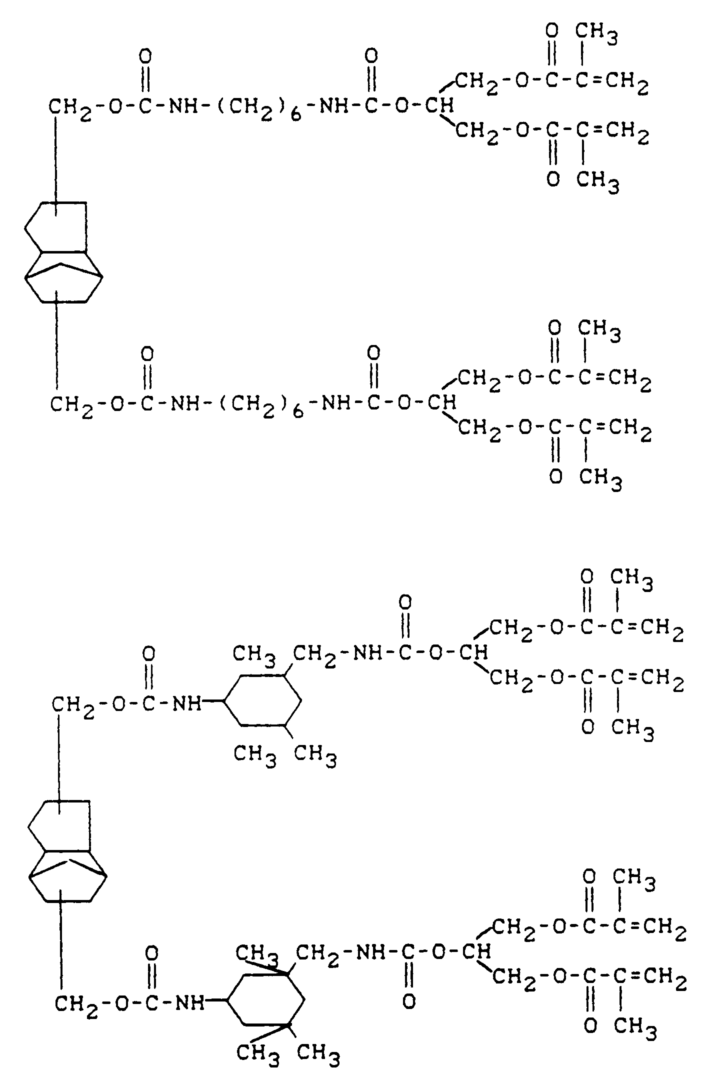 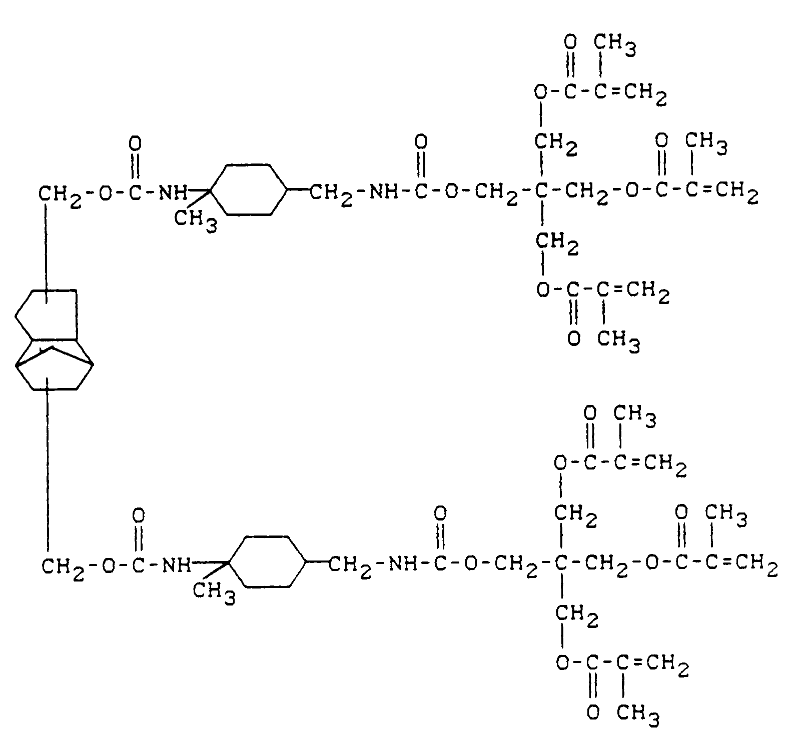 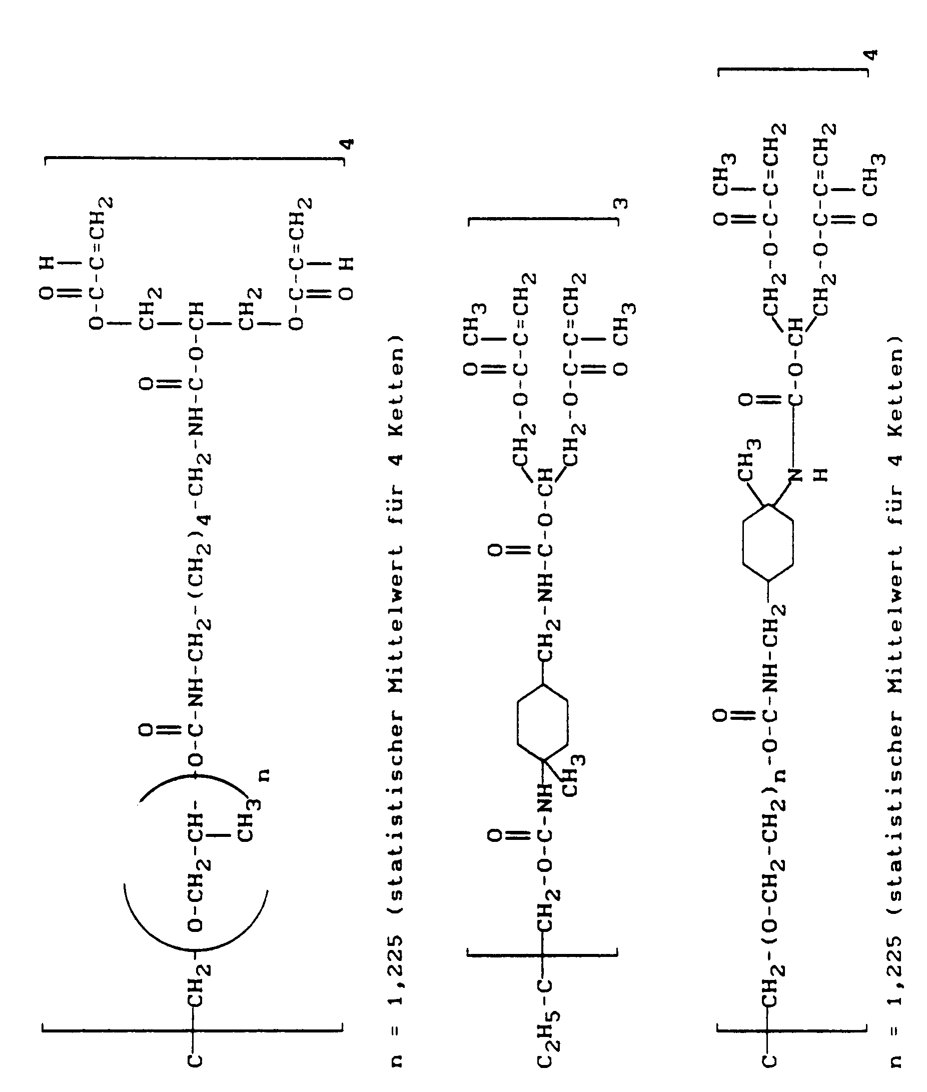 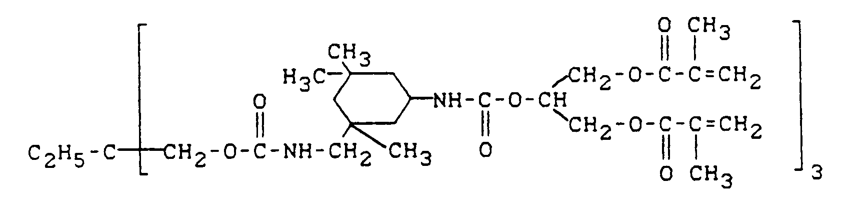
Besonders bevorzugt als Monomer wird das sogenannte Bis-GMA der Formel

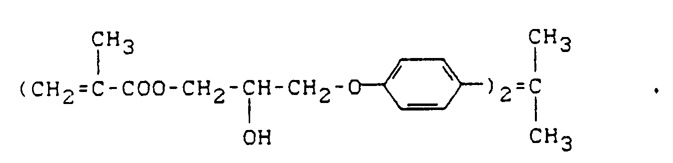

Selbstverständlich ist es möglich, Mischungen der verschiedenen (Meth)-Acrylsäureester, die Vernetzungen ausbilden können, einzusetzen. Beispielsweise seien Mischungen von 20 bis 70 Gew.-Teilen Bis-GMA und 30 bis 80 Gew.-Teilen Triethylenglykoldimethacrylat genannt.

Die erfindungsgemäßen Zubereitungen enthalten im allgemeinen 5 bis 80 Gew.-Teile, bevorzugt 10 bis 60 Gew.-Teile Carboxylverbindungen, bezogen auf die Carbonamidgruppen enthaltenden (Meth)-acrylsäureester.

Als weitere Komponente können die erfindungsgemäßen Zusammensetzungen Füllstoffe enthalten. Als Füllstoffe werden feine Pulver bevorzugt, die einen Teilchendurchmesser im Bereich von 0,1 bis 100 »m (gegebenenfalls auch in einer polydispersen Verteilung) haben. Füllstoffe können im Dentalbereich übliche Füllstoffe (R. S. Baratz, J. Biomat. Applications, Vol 1, 1987, S 316 ff) wie anorganische Gläser, Siliziumdioxid-, Aluminiumoxid-oder Quarzpulver sein.

Durch einen Anteil an Füllstoffen in den erfindungsgemäßen Zubereitungen entstehen Klebezemente, die besonders zur Befestigung von Brücken-, Kronen- und anderen Verblendmaterialen geeignet sind.

Der Anteil des Füllstoffs beträgt im allgemeinen 20 bis 80 Gew.-Teile, bevorzugt 40 bis 70 Gew.- Teile, bezogen auf die gesamte Zubereitung.

Die Adhäsivkomponenten gemäß dieser Erfindung können weiterhin bis zu 10 Gew.-Teilen üblicher Zusätze wie Stabilisatoren, Inhibitoren, Lichtschutzmittel, Farbstoffe, Pigmente oder Fluoreszenzstoffe enthalten.

Die erfindungsgemäßen Zubereitungen können hergestellt werden, indem man den Carbonamidgruppen enthaltenden (Meth)-acrylsäureester und den Initiator und gegebenenfalls die anderen Komponenten durch kräftiges Rühren mischt.

Die Zubereitungen können auch lösungsmittelfrei vorliegen.

Die erfindungsgemäßen Zubereitungen können als Adhäsivkomponente zur Behandlung kollagenhaltiger Materialien verwendet werden.

In einer besonderen Ausführungsform konditioniert man das kollagenhaltige Material vor der Behandlung mit der erfindungsgemäßen Zubereitung mit einer Flüssigkeit mit einem pH-Wert im Bereich von 0,1 bis 3,5.

Diese enthält im allgemeinen Säuren mit einem pKₛ-Wert kleiner als 5 und gegebenenfalls eine amphotere Aminoverbindung mit einem pKₛ-Wert im Bereich von 9,0 bis 10,6 und einem pK_{B}-Wert im Bereich von 11,5 bis 12,5. Folgende Säuren können z.B. in der Konditionierflüssigkeit enthalten sein:
Phosphorsäure, Salpetersäure, Brenztraubensäure, Zitronensäure, Oxalsäure, Ethylendiamintetraessigsäure, Essigsäure, Weinsäure, Äpfelsäure, Maleinsäure.

Als amphotere Aminoverbindungen seien vorzugsweise Verbindungen der Formel 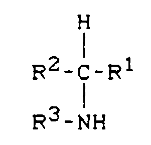
in der
- R¹: für eine Carboxylgruppe steht,
- R²: Wasserstoff, gegebenenfalls durch Hydroxy, Thio, Methylthio, Carboxy, Amino, Phenyl, Hydroxy-phenyl oder die Gruppen 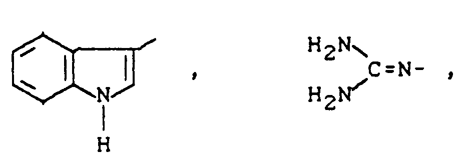 substituierter Niederalkylrest,
- R³: Wasserstoff oder Phenyl bedeutet,
wobei die Reste R¹ und R³ durch einen Propylrest verbunden sein können, oder
in der
- R¹: für Wasserstoff steht,
- R²: für die Gruppe

-A-NH₃X ,

in der
- A: für einen zweibindigen Alkylenrest mit 1 bis 6 Kohlenstoffatomen und
- X: für Halogen steht,
bedeutet und
- R³: Wasserstoff bedeutet,
genannt.

Beispielsweise seien die folgenden amphoteren Aminoverbindungen genannt: Glycin, Serin, Threonin, Cystein, Tyrosin, Asparagin, Glutamin, Alanin, Valin, Leucin, Isoleucin, Prolin, Methionin, Phenylalanin, Tryptophan, Lysin, Arginin, Histidin, N-Phenylglycin, Ethylendiaminhydrochlorid, Ethylendiaminhydrobromid, Propylendiaminhydrochlorid, Propylendiaminhydrobromid, Butylendiaminhydrochlorid, Butylendiaminhydrobromid, Leucinhydrochlorid und Histidinhydrochlorid.

Weiterhin kann die Konditionierflüssigkeit Stoffe aus der Gruppe der Polyethylenglykole und Metallhydroxide enthalten. Insbesondere können die oben aufgeführten mehrbasigen Säuren auch als teilweise Metallsalze eingesetzt werden solange freie Säurefunktionen verbleiben.

Konditionierflüssigkeiten, die mindestens eine der Säuren aus der Gruppe der Brenztraubensäure, Ethylendiamintetraessigsäure und Zitronensäure sowie gegebenenfalls eine amphotere Aminoverbindung aus der Gruppe des Glycins, N-Phenylglycins und Prolins enthalten, sind besonders bevorzugt.

Die Anwendung der erfindungsgemäßen Zubereitungen kann beispielsweise wie folgt durchgeführt werden:
Bei einer Zahnreparatur trägt man beispielsweise nach einer mechanischen Reinigung des kollagenhaltigen Zahnmaterials zuerst die Konditionierflüssigkeit mit etwas Watte auf, läßt eine kurze Zeit (beispielsweise 60 Sekunden) einwirken, spült das Zahnmaterial mit Wasser und trocknet es im Luftstrom. Dann trägt man die erfindungsgemäße Zubereitung beispielsweise mit einem kleinen Pinsel in einer dünnen Schicht auf und trocknet im Luftstrom. Nach der erfindungsgemäßen Behandlung trägt man die eigentliche Füllmasse, beispielsweise im Dentalbereich übliche Kunststoffüllungsmassen (K. Eichner, "Zahnärztliche Werkstoffe und ihre Verarbeitung", Bd. 2, S. 135 ff, Hüthig Verlag, 5. Aufl. 1985) auf.

In ähnlicher Weise können die erfindungsgemäßen Zubereitungen zur Befestigung von Kronen, Brücken und ähnlichen Hilfsmitteln verwendet werden.

### Beispiel 1: Synthese der neuen Carbonamidgruppen enthaltenden (Meth)acrylsäureester (I)

1a) Methacrylsäure-3-acetamidopropylesters
   Vorstufe:
   Zu 300,4 g (4,00 mol) 3-Aminopropanol in 600 ml Methanol wurden unter Rühren bei 25°C 290,3 g (4,00 mol) Essigsäuremethylester getropft und anschließend 5 Stunden zum Rückfluß erhitzt. Nach Einengen des Ansatzes wurden bei 0,3 Torr und 137-140°C 328,0 g (70 % der Theorie) an N-(3-Hydroxypropyl)-acetamid in Form einer farblosen Flüssigkeit erhalten.
   Folgestufe:
   Zu einer auf -72°C gekühlten Vorlage bestehend aus 206,2 g (1,76 mol) N-(3-Hydroxypropyl)-acetamid, 750 ml Methylenchlorid, 228 g (2,25 mol) Triethylamin und 250 mg 2,6-Di-tert.butyl-4-methylphenol wurden während 2 Stunden 184,0 g (1,76 mol) Methacrylsäurechlorid getropft. Es wurde 2 weitere Stunden bei -52°C gerührt und der ausgefallene helle Niederschlag anschließend bei 0°C abgesaugt. Das Filtrat wurde wäßrig aufgearbeitet, getrocknet und nach dem Einengen am Dünnschichter zuerst bei 100°C, dann der Rückstand bei 130°C (jeweils 0,1 Torr) destilliert. Es wurden 174,1 g (53 % der Theorie) des Methacrylsäure-3-acetamidopropylesters in Form eines farblosen Öles erhalten.
   - IR (Film):: γ = 3280, 2930, 1712, 1640, 1542, 1439, 1365, 1320, 1295, 1160, 938, 810 cm⁻¹
   - ¹H-NMR: (CDCl₃, 200 MHz): δ = 1,90 (m, 2H, C-CH₂-C), 1,95 (bs, 3H, COCH₃), 1,99 (bs, 3H, vinyl.-CH₃), 3,33 (m, 2H, NCH₂), 4,24 (t, J=6Hz, 2H, OCH₂), 5,60, 6,12 (2bs, je 1H, vinyl.-H), 5,95 (bs, 1H, NH)
   MS (70 eV): m/z = 185 (M⁺), 99 (M-C₃H₅CO₂H), 69 (C₃H₅CO⁺), 57 (C₃H₅O⁺), 43 (CONH⁺), 41 (C₃H₅⁺), 30.
   Andere erfindungsgemäße (Meth)acrylsäureester (I) lassen sich entsprechend darstellen. Als ein Beispiel für ein erfindungsgemäßes Propionamid sei der Methacrylsäure-3-propionamidopropylester mit folgenden spektroskopischen Daten genannt:
   - IR (Film):: γ = 3280, 3060, 2940, 2940, 1720, 1640, 1540, 1480, 1318, 1296, 1160, 1044, 932, 807 cm⁻¹
   - ¹H-NMR: (CDCl₃, 200 MHz): δ=1,18 (t, J = 7,5 Hz, 3H, CH₂CH₃), 1,89 (m, 2H, CH₂CH₂CH₂), 1,96 (bs, 3H, COCCH₃), 2,23 (q, J = 7,5 Hz, 2H, CH₂CH₃), 3,34 (m, 2H, NCH₂), 4,22 (t, J = 6,2 Hz, 2H, OCH₂), 5,6, 6,1 (2 bs, je 1H, vinyl.-H), 6,19 (bs, 1H, NH)ppm.
1b) N-3-Methacryloyloxypropylmethacrylamid
   Zu einer Vorlage aus 75,1 g (1,00 mol) 3-Aminopropanol, 404,4 g (4,00 mol) Triethylamin und 100 mg 2,6-Di-tert.butyl-4-methylphenol in 400 ml Methylenchorid wurden unter Rühren bei -35°C 205,0 g (2,00 mol) Methacrylsäurechlorid getropft und zwei Stunden auf -30°C gehalten. Nach Absaugen des entstandenen Niederschlages und wäßriger Extraktion wurde die organische Phase zu 122,3 g (58 % der Theorie) Produkt eingeengt, das nach Eluieren mit Ether/n-Hexan (6:4) von einer Kieselgelsäule als farbloses Öl vorlag.
   - IR (Film):: γ = 3340, 3080, 2970, 1723, 1663, 1623, 1539, 1456, 1321, 1300, 1168, 1010, 940, 816 cm⁻¹
   - ¹H-NMR: (CDCl₃, 360 MHz): δ = 1,94 (m, 2H, CH₂CH₂CH₂), 1,98, 2,00 (2s, je 3H, CH₃), 3,41 (m, 2H, NCH₂), 4,26 (t, J = 6,5 Hz, 2H, OCH₂), 5,35, 5,60, 5,72, 6,13 (4 bs, je 1H, vinyl.-H), 6,33 (bs, 1H, NH)ppm.
   - MS (70 eV):: m/z = 211 (M⁺), 142 (M-C₃H₅CO), 69 (C₃H₅CO⁺), 41 (C₃H₅⁺).

### Beispiele 2 bis 9: Herstellung der Zubereitungen

Die erfindungsgemäßen Klebemittel werden durch intensives Vermischen der in folgenden Beispielen aufgeführten Bestandteile erzeugt.
- Beispiel 2:: 54 g Wasser
46 g Methacrylsäure-2-acetamidoethylester
138 mg Campherchinon
- Beispiel 3:: 39 g Wasser
41 g Methacrylsäure-2-acetamidoethylester
20 g 25 Gew.-%ige wäßrige Glutardialdehyd-Lösung
123 mg Campherchinon
- Beispiel 4:: 52 g Wasser
48 g Methacrylsäure-3-acetamidopropylester
144 mg Campherchinon
48 mg Propionaldehyd
- Beispiel 5:: 40 g Wasser
44 g Methacrylsäure-3-acetamidopropylester
16 g 25 Gew.-%ige wäßrige Glutardialdehyd-Lösung
132 mg Campherchinon
- Beispiel 6:: 52 g Wasser
48 g Methacrylsäure-3-propionamidopropylester
144 mg Campherchinon
- Beispiel 7:: 37 g Wasser
43 g Methacrylsäure-3-propionamidopropylester
20 g 25 Gew.-%ige, wäßrige Glutardialdehyd-Lösung
129 mg Campherchinon
- Beispiel 8:: 33 g Wasser
34 g N-3-Methacryloyloxypropylmethacrylamid
102 mg Campherchinon
- Vergleichsbeispiel 9:: 54 g Wasser
46 g Methacrylsäure-5-propionamidopentylester
138 mg Campherchinon

### Beispiel 10: (Anwendung)

Die Eignung der Klebemittel entsprechend der Beispiele 1 bis 9 wird geprüft, indem die Bindungsfestigkeit der lichtaktivierten Kunststoffüllungsmasse auf Basis mehrfunktioneller Methacrylsäureester und Bariumaluminosilikat Lumifor® auf Dentin bestimmt wird, das nacheinander mit der Konditionierflüssigkeit (bestehend aus 81,2 g Wasser, 1,7 g Natriumhydroxid und 17 g Ethylendiamintetraessigsäure-Dinatrium-Dihydrat: 60 Sekunden Einwirkzeit, Wasserspülung, Lufttrocknung), dem Klebemittel (60 Sekunden Einwirkzeit, Lufttrocknung) und einem Verschlußmaterial auf Basis polyfunktioneller Methacrylsäureester (Bayer Resin L®) (Auftragen und im Luftstrom dünn verteilen) vorbehandelt worden ist.

Für den Test werden herausgezogene und im feuchten Zustand aufbewahrte Menschenzähne benutzt. Die Zähne werden durch Guß in Epoxidharz eingelagert; durch Nachschleifen wird eine glatte Dentinoberfläche erzeugt. Das anschließende Schleifen erfolgt mit Carbonpapier 1000.

Zur Herstellung eines Probekörpers zum Messen der Bindungsstärke wird eine zylindrische, gespaltete Teflonform auf die wie vorstehend beschrieben behandelte Dentinoberfläche gespannt (Scand. J. Dent. Res. 88, 348-351 (1980)). Als Füllmasse wird ein handelsübliches Kunststoffüllungsmaterial eingefüllt. Ein in einem Loch in einer Bohrerhalterung eingespannter Rundbohrer Nr. 016 wird an der Teflonform befestigt und von oben in die noch im Härteprozeß befindliche Materialschicht gepreßt.

Die gesamte Anordnung wird 10 Minuten lang bei Zimmertemperatur (25°C) ungestört stehengelassen, wonach die Bohrerhalterung und die Teflonform abgenommen und die Probe unter Wasser mit einer Temperatur von 23°C abgesetzt wird. Nach 15 Minuten wird die Probe mit dem Bohrer in eine Instron-Zugversuchsapparatur (Scand. J. Dent. Res. 88, 348-351 (1980)) montiert; mit einer Geschwindigkeit von 1 mm/Min. wird eine Zugfestigkeitsmessung durchgeführt. Die Zugfestigkeit errechnet sich aus der Division der beim Bruch der Füllung angelegten Belastung mit dem Querschnittsareal in der Bruchfläche des Probekörpers. Es wurden jeweils 5 Messungen an Probekörpern durchgeführt.

Die Ergebnisse sind in der folgenden Tabelle zusammengefaßt:

| Zubereitung nach Beispiel Nr. | Zugbindungsfestigkeit [N/mm²] |
|---|---|
| 2 | 19 ± 2 |
| 3 | 21 ± 3 |
| 4 | 14 ± 2 |
| 5 | 18 ± 3 |
| 6 | 8 ± 2 |
| 7 | 12 ± 4 |
| 8 | 6 ± 2 |
| 9 | 2 ± 1 |

## Patentansprüche (Patentansprüche für folgende(n) Vertragsstaat(en): AT, BE, CH, DE, DK, FR, GB, GR, IT, LI, LU, NL, SE)

1. Zubereitungen, enthaltend Carbonamidgruppen enthaltende (Meth)acrylsaureester (I) der Formel (I) 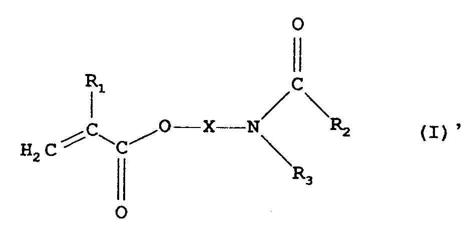 in der
R₁ Wasserstoff oder Methyl bedeutet,
R₂ gegebenenfalls durch Hydroxy, Carboxy, Halogen oder Amino der Formel 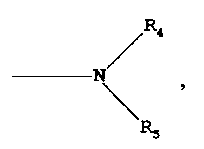 in der
R₄ und R₅ gleich oder verschieden sind und Wasserstoff oder Niederalkyl bedeuten,
substituiertes Alkyl (C₁-C₄) oder Alkenyl (C₂-C₄) bedeutet,
R₃ Wasserstoff bedeutet oder eine der oben für R₂ genannten Bedeutungen hat und
X ein zweiwertiger, gegebenenfalls durch Hydroxy, Carboxy, Halogen oder Amino der Formel 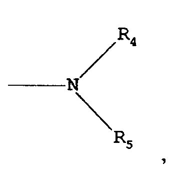 in der
R₄ und R₅ die oben genannte Bedeutung haben,
substituierter aliphatischer (C₁-C₆) oder cycloaliphatischer Rest (C₃-C₆), der gegebenenfalls eine oder mehrere Sauerstoff-, Schwefel- und/oder NR₄-Brücken enthalten kann, wobei
R₄ die oben genannte Bedeutung hat,
ist, mit der Maßgabe, daß
a) die Summe der Kohlenstoffatome in den Resten R₂, R₃ und X nicht größer als sechs ist, sofern diese Reste keine Heteroatome enthalten, oder
b) die Summe der Kohlenstoffatome in den Resten R₂, R₃, R₄, R₅ und X nicht größer als zehn ist, sofern mindestens einer dieser Reste zumindest ein Heteroatom enthält,
und gegebenenfalls Initiatoren
als Adhäsivkomponente zur Behandlung kollagenhaltiger Materialien zur Anwendung in der Medizin.

2. Zubereitung nach Anspruch 1, enthaltend Carbonamidgruppen enthaltende (Meth)acrylsäureester, worin
R₁ Wasserstoff oder Methyl bedeutet,
R₂ gegebenenfalls durch Hydroxy, Carboxy, Fluor, Chlor oder Amino substituiertes Alkyl (C₁-C₃) bedeutet,
R₃ Wasserstoff bedeutet oder eien der für R₂ genannten Bedeutungen hat und
X einen zweiwertigen, gegebenenfalls durch Hydroxy, Carboxy, Fluor, Chlor oder Amino substituierten, aliphatischen (C₁-C₆) und/oder cycloaliphatischen (C₃-C₆) Rest, der gegebenenfalls ein bis fünf Sauerstoff- oder -NR₄-Brücken enthalten kann, wobei
R₄ die oben genannte Bedeutung hat,
bedeutet.

3. Zubereitungen nach den Ansprüchen 1 und 2, dadurch gekennzeichnet, daß als Initiator ein Radikalbildner aus der Reihe der Mono- oder Dicarbonylverbindungen eingesetzt wird.

4. Zubereitung nach den Ansprüchen 1 bis 3, dadurch gekennzeichnet, daß ein Coaktivator zugesetzt wird.

5. Zubereitung nach den Ansprüchen 1 bis 4, dadurch gekennzeichnet, daß als weitere Komponente eine Carbonylverbindung zugesetzt wird.

6. Zubereitung nach den Ansprüchen 1 bis 5, dadurch gekennzeichnet, daß als weitere Komponente (Meth)acrylsäureester, die Vernetzungen ausbilden können, zugesetzt werden.

## Patentansprüche (Patentansprüche für folgende(n) Vertragsstaat(en): ES)

1. Verfahren zur Herstellung von Zubereitungen enthaltend Carbonamidgruppen enthaltende (Meth)acrylsäureester (I) der Formel (I) 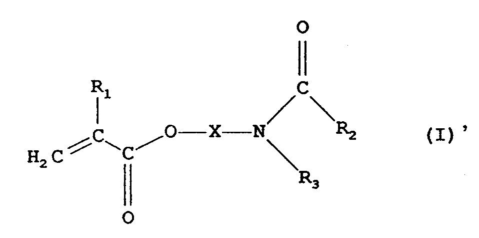 in der
R₁ Wasserstoff oder Methyl bedeutet,
R₂ gegebenenfalls durch Hydroxy, Carboxy, Halogen oder Amino der Formel 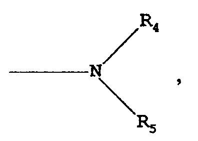 in der
R₄ und R₅ gleich oder verschieden sind und Wasserstoff oder Niederalkyl bedeuten,
substituiertes Alkyl (C₁-C₄) oder Alkenyl (C₂-C₄) bedeutet,
R₃ Wasserstoff bedeutet oder eine der oben für R₂ genannten Bedeutungen hat und
X ein zweiwertiger, gegebenenfalls durch Hydroxy, Carboxy, Halogen oder Amino der Formel 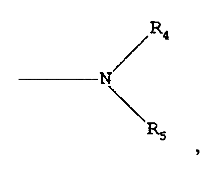 in der
R₄ und R₅ die oben genannte Bedeutung haben,
substituierter aliphatischer (C₁-C₆) oder cycloaliphatischer Rest (C₃-C₆), der gegebenenfalls eine oder mehrere Sauerstoff-, Schwefel- und/oder NR₄-Brücken enthalten kann, wobei
R₄ die oben genannte Bedeutung hat,
ist, mit der Maßgabe, daß
a) die Summe der Kohlenstoffatome in den Resten R₂, R₃ und X nicht größer als sechs ist, sofern diese Reste keine Heteroatome enthalten, oder
b) die Summe der Kohlenstoffatome in den Resten R₂, R₃, R₄, R₅ und X nicht größer als zehn ist, sofern mindestens einer dieser Reste zumindest ein Heteroatom enthält,
dadurch gekennzeichnet, daß man (Meth)acrylsäurechlorid (Ia) 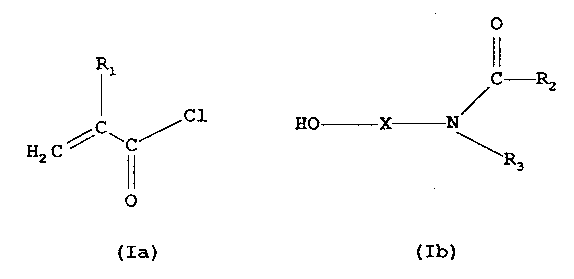 mit Alkanolaminen (Ib), in denen R₁, R₂, R₃ und X die oben genannte Bedeutung haben, umsetzt, die erhaltenen (Meth)acrylsäureester isoliert und durch deren Abmischung mit gegebenenfalls Initiatoren, Lösungsmitteln, Coaktivatoren, Carbonylverbindungen, zur Ausbildung von Vernetzungen geeigneten (Meth)-acrylsäureestern, Füllstoffen und bis zu 10 Gew.-Teilen, bezogen auf die Gesamtmischung, an üblichen Zusätzen die Zubereitung zur Behandlung kollagenhaltiger Materialien in der Medizin erhält.

## Claims (Claims for the following Contracting State(s): AT, BE, CH, DE, DK, FR, GB, GR, IT, LI, LU, NL, SE)

1. Preparations, containing carboxamide group-containing (meth)acrylic acid eaters (I) of the formula (I) 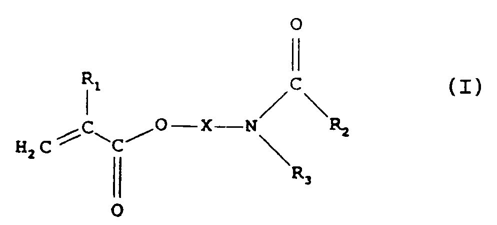 in which
R₁ denotes hydrogen or methyl,
R₂ denotes alkyl (C₁-C₄) or alkenyl (C₂-C₄), optionally substituted by hydroxyl, carboxyl, halogen or amino of the formula 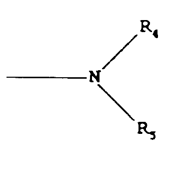
in which
R₄ and R₅ are identical or different and denote hydrogen or lower alkyl,
R₃ denotes hydrogen or has one of the abovementioned meanings of R₂ and
X is a divalent aliphatic (C₁-C₆) or cycloaliphatic radical (C₃-C₆), which is optionally substituted by hydroxyl, carboxyl, halogen or amino of the formula 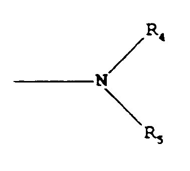
in which
R₄ and R₅ have the abovementioned meaning,
and can optionally contain one or more oxygen, sulphur and/or NR₄ bridges,
where
R₄ has the abovementioned meaning,
with the proviso that
a) the sum of the carbon atoms in the radicals R₂, R₃ and X is not greater than six if these radicals contain no heteroatoms, or
b) the sum of the carbon atoms in the radicals R₂, R₃, R₄, R₅ and X is not greater than ten if at least one of the radicals contains at least one heteroatom,
and, if appropriate, initiators,
as adhesive component for the treatment of collagen-containing materials for use in medicine.

2. Preparation according to Claim 1, containing carboxamide group-containing (meth)acrylic acid esters, in which
R₁ denotes hydrogen or methyl,
R₂ denotes alkyl (C₁-C₃) optionally substituted by hydroxyl, carboxyl, fluorine, chlorine or amino,
R³ denotes hydrogen or has one of the meanings mentioned for R₂ and
X denotes a divalent aliphatic (C₁-C₆) and/or cycloaliphatic (C₃-C₆) radical, which is optionally substituted by hydroxyl, carboxyl, fluorine, chlorine or amino and can optionally contain one to five oxygen or -NR₄ bridges, where
R₄ has the abovementioned meaning.

3. Preparations according to Claims 1 and 2, characterized in that a free radical former from the series comprising the mono- or dicarbonyl compounds is employed as initiator.

4. Preparation according to Claims 1 to 3, characterized in that a coactivator is added.

5. Preparation according to Claim 1 to 4, characterized in that a carbonyl compound is added as further component.

6. Preparation according to Claims 1 to 5, characterized in that (meth)acrylic acid esters which can form cross-linkages are added as further component.

## Claims (Claims for the following Contracting State(s): ES)

1. Process for the production of preparations containing carboxamide group-containing (meth)acrylic acid esters (I) of the formula (I) 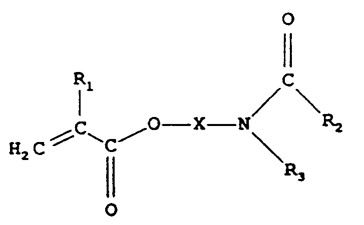 in which
R₁ denotes hydrogen or methyl,
R₂ denotes alkyl (C₁-C₄) or alkenyl (C₂-C₄), optionally substituted by hydroxyl, carboxyl, halogen or amino of the formula 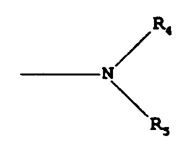
in which
R₄ and R₅ are identical or different and denote hydrogen or lower alkyl,
R₃ denotes hydrogen or has one of the abovementioned meanings of R₂ and
X is a divalent aliphatic (C₁-C₆) or cycloaliphatic radical (C₃-C₆), which is optionally substituted by hydroxyl, carboxyl, halogen or amino of formula 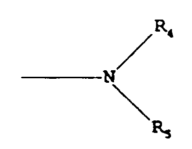
in which
R₄ and R₅ have the abovementioned meaning,
and can optionally contain one or more oxygen, sulphur and/or NR₄ bridges,
where
R₄ has the abovementioned meaning,
with the proviso that
a) the sum of the carbon atoms in the radicals R₂, R₃ and X is not greater than six if these radicals contain no heteroatoms, or
b) the sum of the carbon atoms in the radicals R₂, R₃, R₄, R₅ and X is not greater than ten if at least one of these radicals contains at least one heteroatom,
characterized in that (meth)acryloyl chloride (Ia) is 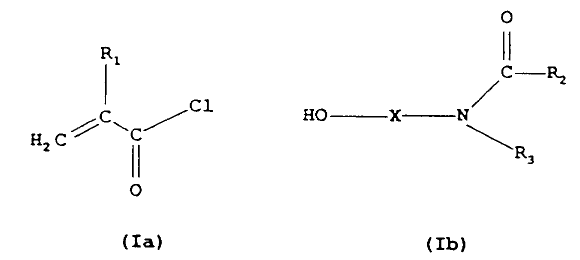 reacted with alkanolamines (Ib) in which R₁, R₂, R₃ and X have the abovementioned meaning, the resulting (meth)acrylic acid esters are isolated, and by admixture thereof with, optionally, initiators, solvents, coactivators, carbonyl compounds, (meth)acrylic acid esters suitable for the formation of cross-linkages, fillers and up to 10 parts by weight, relative to the complete mixture, of customary additives the preparation for the treatment of collagen-containing materials in medicine is obtained.

## Revendications (Revendications pour l'(les) Etat(s) contractant(s) suivant(s): AT, BE, CH, DE, DK, FR, GB, GR, IT, LI, LU, NL, SE)

1. Préparations contenant des esters (méth)acryliques (I) de formule (I) contenant des groupes carboxamide 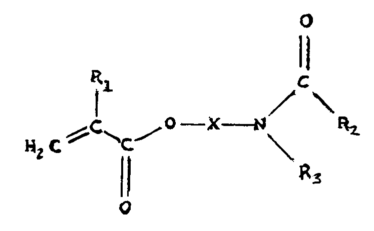 dans laquelle
R¹ représente un atome d'hydrogène ou un groupe méthyle,
R² représente un groupe alkyle en C₁-C₄ ou un groupe alcényle en C₂-C₄ éventuellement substitué par un groupe hydroxyle, par un groupe carboxyle, par un atome d'halogène ou par un groupe amino de formule 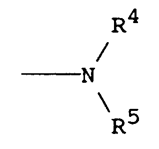
dans laquelle
R⁴ et R⁵ sont identiques ou différents et représentent un atome d'hydrogène ou un groupe alkyle inférieur,
R³ représente un atome d'hydrogène ou bien a une des significations mentionnées ci-dessus pour R², et
X est un radical bivalent aliphatique en C₁-C₆ ou cycloaliphatique en C₃-C₆ éventuellement substitué par un groupe hydroxyle, par un groupe carboxyle, par un atome d'halogène ou par un groupe amino de formule 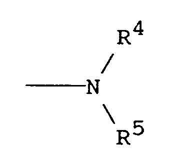 dans laquelle
R⁴ et R⁵ ont la signification mentionnée ci-dessus,
qui peut éventuellement contenir un ou plusieurs ponts oxygène, soufre et/ou NR⁴, où
R⁴ a la signification mentionnée ci-dessus,
avec cette mesure que
a) la somme des atomes de carbone dans les radicaux R², R³ et X n'est pas supérieure à six pour autant que ces radicaux ne contiennent pas d'hétéroatomes, ou
b) la somme des atomes de carbone dans les radicaux R², R³, R⁴, R⁵ et X n'est pas supérieure à dix pour autant qu'au moins un de ces radicaux contienne au moins un hétéroatome,
et éventuellement des initiateurs,
comme composant adhésif pour le traitement de matières contenant du collagène pour une application dans la médecine.

2. Préparation selon la revendication 1, contenant des esters (méth)acryliques contenant des groupes carboxamide, dans laquelle
R¹ représente un atome d'hydrogène ou un groupe méthyle,
R² représente un groupe alkyle en C₁-C₃ éventuellement substitué par un groupe hydroxyle, par un groupe carboxyle, par un atome de fluor, par un atome de chlore ou par un groupe amino,
R³ représente un atome d'hydrogène ou a une des significations mentionnées pour R², et
X représente un radical bivalent aliphatique en C₁-C₆ et/ou cycloaliphatique en C₃-C₆ éventuellement substitué par un groupe hydroxyle, par un groupe carboxyle, par un atome de fluor, par un atome de chlore ou par un groupe amino, qui peut éventuellement contenir de 1 à 5 ponts oxygène ou NR⁴ où
R⁴ a la signification mentionnée ci-dessus.

3. Préparations selon les revendications 1 et 2, caractérisées en ce qu'on met en oeuvre, comme initiateur, un formateur de radicaux de la série des composés mono- ou dicarbonyle.

4. Préparation selon les revendications 1 à 3, caractérisée en ce qu'un coactivateur est ajouté.

5. Préparation selon les revendications 1 à 4, caractérisée en ce que, comme autre composant, est ajouté un composé carbonyle.

6. Préparation selon les revendications 1 à 5, caractérisée en ce que, comme autre composant, sont ajoutés des esters (méth)acryliques qui peuvent former des réticulations.

## Revendications (Revendications pour l'(les) Etat(s) contractant(s) suivant(s): ES)

1. Procédé pour la mise au point de préparations contenant des esters (méth)acryliques (I) de formule (I) contenant des groupes carboxamide 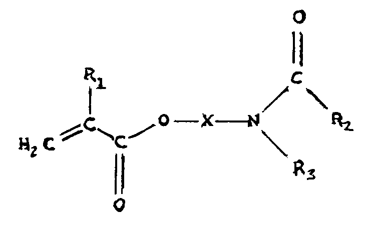 dans laquelle
R¹ représente un atome d'hydrogène ou un groupe méthyle,
R² représente un groupe alkyle en C₁-C₄ ou un groupe alcényle en C₂-C₄ éventuellement substitué par un groupe hydroxyle, par un groupe carboxyle, par un atome d'halogène ou par un groupe amino de formule 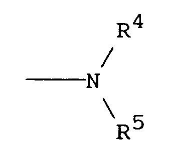
dans laquelle
R⁴ et R⁵ sont identiques ou différents et représentent un atome d'hydrogène ou un groupe alkyle inférieur,
R³ représente un atome d'hydrogène ou bien a une des significations mentionnées ci-dessus pour R², et
X est un radical bivalent aliphatique en C₁-C₆ ou cycloaliphatique en C₃-C₆ éventuellement substitué par un groupe hydroxyle, par un groupe carboxyle, par un atome d'halogène ou par un groupe amino de formule 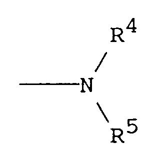 dans laquelle
R⁴ et R⁵ ont la signification mentionnée ci-dessus,
qui peut éventuellement contenir un ou plusieurs ponts oxygène, soufre et/ou NR⁴, où
R⁴ a la signification mentionnée ci-dessus,
avec cette mesure que
a) la somme des atomes de carbone dans les radicaux R², R³ et X n'est pas supérieure à six pour autant que ces radicaux ne contiennent pas d'hétéroatomes, ou
b) la somme des atomes de carbone dans les radicaux R², R³, R⁴, R⁵ et X n'est pas supérieure à dix pour autant qu'au moins un de ces radicaux contienne au moins un hétéroatome,
caractérisé en ce qu'on fait réagir du chlorure d'acide (méth)acrylique (Ia) 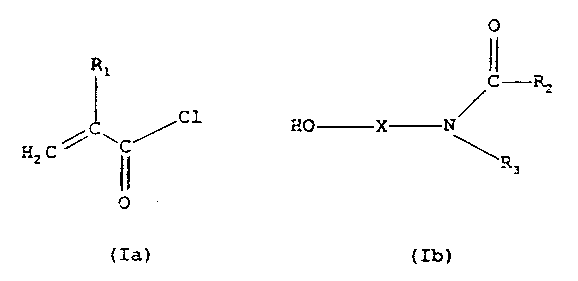 avec des alcanolamines (Ib), dans lesquelles R¹, R², R³ et X ont la signification mentionnée ci-dessus, on isole les esters (méth)acryliques obtenus et, par leur mélange avec éventuellement des initiateurs, des solvants, des coactivateurs, des composés carbonyle, des esters (méth)acryliques appropriés pour la formation de réticulation, des matières de charge et jusqu'à 10 parties en poids, rapportées sur le mélange total, d'additifs habituels, on obtient la préparation destinée au traitement dans la médecine de matières contenant du collagène.
